(19) Europäisches Patentamt
European Patent Office
Office européen des brevets

(11) Publication number: **0 262 746 B1**

# (12) EUROPEAN PATENT SPECIFICATION

(45) Date of publication of patent specification: **12.05.93**

(51) Int. Cl.[5]: **C08L 63/02**, C08L 9/00, C08G 59/50, C08G 59/24, //(C08L63/02,9:00)

(21) Application number: **87201883.3**

(22) Date of filing: **01.10.87**

## (54) Thermosettable resin composition comprising a polydiene elastomer.

(30) Priority: **03.10.86 US 915217**

(43) Date of publication of application:
**06.04.88 Bulletin 88/14**

(45) Publication of the grant of the patent:
**12.05.93 Bulletin 93/19**

(84) Designated Contracting States:
**BE DE ES FR GB IT NL SE**

(56) References cited:
**EP-A- 0 203 828**
**US-A- 4 593 056**

**Epoxy Resins and Compositions I, K. Dusek, A.J. Kinloch, p. 50-56 (1985), Springer Verlag, New York,**

**EPOXY Resins, Ed.: C.A. May, Y. Tanaka, p. 506, 516 (1973), Marcel Dekker, Inc. New York**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **De La Mare, Harold Elison**
**42 Chee Hoon**
**Singapore 1129(SG)**
Inventor: **Allen, Robert Charles**
**8515 Cookwind**
**Houston Texas 77072(US)**
Inventor: **Bauer, Ronald Sherman**
**5427 Crown Colony**
**Houston Texas 77069(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 262 746 B1

## Description

Thermosettable resin compositions, such as amine-cured epoxies, are well known for structural applications requiring strength, water resistance and toughness.

United States patent No. 4,593,056 relates to an epoxy/aromatic diamine system, comprising an aromatic trihydroxy accelerator in order to improve the cure speed of said system. From "Epoxy resins and composites I", K. Dusek, A.J. Kinloch, pages 50-56 (1985) Springer-Verlag Berlin, Heidelberg, it is known that multiphase microstructures can be established of simple epoxy polymers (e.g. the diglycidyl ether of bisphenol-A) and of rubbery particles (e.g. a carboxyl terminated butadiene-acrylonitrile copolymer), which when cured with an amine hardener show a higher fracture energy in the cured state, when compared with the same epoxy resin/amine system without the rubbery dispersed phase.

Strong but lightweight materials are needed for increasingly demanding applications such as aerospace structural composites. For aerospace applications, it is necessary to have thermosettable materials which have high glass transition temperatures, good fracture toughness and low water absorption at high temperature.

It is therefore an object of the invention to provide new thermosetting resin compositions having properties suitable for aerospace and other high performance applications.

According to the invention, a composition is provided comprising

(a) a diglycidyl ether of 9,9-bis(4-hydroxyphenyl)fluorene;

(b) normally liquid elastomeric polydiene comprising carboxyl and/or amine groups and having a molecular weight of from 2000 to 10000, and

(c) a polyaromatic polyamine curing agent for the diglycidyl ether. The polyaromatic polyamine curing agent is present in an amount of at least 20 per cent stoichiometric excess with respect to the diglycidyl ether.

The diglycidyl ethers of 9,9-bis(4-hydroxyphenyl)fluorene can be prepared by glycidating the corresponding bisphenol as is known in the art and described, for example, by Lin and Pearce in J. Polym. Sci., Polym. Chem. Ed. 17, 3095-3119.

Component (b) of the invention composition is an elastomeric material which contains reactive groups such as $-CO_2H$ or $NH_2$. Preferred are carboxy- and amine-terminated polydiene rubbers. The term "normally liquid" refers to being liquid at room temperature. Polydiene rubbers are typically prepared by solution polymerization, in the presence of a lithium catalyst, of one or more conjugated diene monomers having from 4 to 12 carbon atoms. Typical diene monomers include 1,3-butadiene, isoprene, piperylene and methyl-pentadiene. Also included within the scope of the rubber components are carboxy- and amine-terminated liquid copolymers of a conjugated diene and a copolymerizable vinyl monomer. Suitable copolymerizable monomers include alkenyl aromatics such as styrene, vinyl toluene and $\alpha$-methyl styrene; nitriles such a acrylonitrile and methacrylonitrile; acrylate and methacrylate esters; and the like. The copolymers will generally contain at least 40 weight per cent of one or more dienes, generally at least 60 weight per cent, and 0 to 60 weight per cent of one or more copolymerizable vinyl monomers. Preferred molecular weights are from 3000 to 8000. Preferred elastomer components for the invention composition include carboxy-terminated butadiene/acrylonitrile rubbers available under the tradename HyCar (HyCar is a registered tradename) reactive liquid polymers manufactured by B.F. Goodrich Company.

Component (b) is present in the composition in an amount of 3 to 40, preferably 5 to 25, most preferably 10 to 20 weight per cent, based on the weight of the diglycidyl ether.

The polyaromatic polyamine component of the invention is a curing agent having at least two aromatic rings and at least two primary or secondary amine groups per molecule. Such polyaromatic polyamine curing agents include aromatic amines of the formula

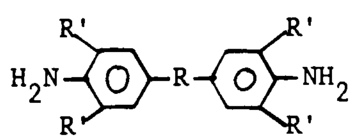
,

in which R is an aromatic moiety, $-CH_2-$, $C(CH_3)_2$, $-O-$, $-SO_2$, $-S-$ or

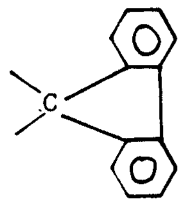

,

and each R' is independently selected from H, lower alkyl including methyl and ethyl, and halide. Such polyaromatic polyamine curing agents are available from Shell Chemical Company under the tradenames EPON HPT Curing Agents 1061 and 1062 (EPON HPT is a registered tradename). The preferred polyaromatic polyamine curing agents can be described by the formula in which R is

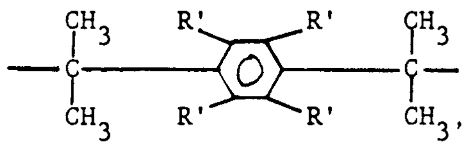

,

particularly when used in combination with the described polyaromatic polyamine curing agent in which R is $-SO_2-$ to provide a combination of good phase separation between the rubber and diglycidyl ether and acceptably low water absorbance during cure. Such a curing agent mixture is most effective when the sulfone is present in the curing agent mixture in an amount of from 15 to 65 weight per cent, preferably 30 to 60 weight per cent.

The amine component of the composition is present in at least a 20 per cent stoichiometric excess with respect to the diglycidyl ether. Preferably, the amine will be present in the composition in an amount of from 1.2 to 1.8 equivalents per equivalent of the diglycidyl ether, most preferably from 1.25 to 1.5 equivalents.

The invention composition can be prepared by mixing the three components, preferably in the melt with stirring or agitation. The preferred method for preparing the composition is to melt the diglycidyl ether resin at a temperature of from 110 to 150 °C; dissolve the elastomer in the molten resin; introduce a suitable adduction or transesterification catalyst such a triphenyl phosphine ethyl iodide or sodium hydroxide, for example, to produce a resin/rubber adduct; hold the adduct at about 150 °C for about 1 to 3 hours; add the resin curing agent with stirring or agitation; and pour the resulting mixture into a mold or onto the desired substrate and cure for about 2 to 6 hours at a temperature of about 140 to 200 °C.

The composition optionally, but preferably for high-performance applications such as automotive and aerospace structural parts, contains reinforcing substrate. Suitable reinforcing materials include, for example, glass fibers, carbon fibers, polyaramide, boron, calcium carbonate, talc, alumina, asbestos and the like. The preferred fibrous reinforcing material for high-performance applications is continuous carbon fiber. The fibrous reinforcing material will be present in the composition in an amount effective to impart increased strength to the cured composition, generally from 40 to 95 weight per cent, usually from 60 to 80 weight per cent, based on the weight of the total composition.

The diglycidyl ether can be applied to the fibrous reinforcing material from the melt or solution by methods known in the art. The diglycidyl ether/curing agent-impregnated substrate, or "prepreg," or laminate prepared from a plurality of prepregs, is then cured, generally at a temperature of 160 °C to 300 °C for 30 minutes to 4 hours and a pressure of 160 to 240 psi, to form the structural composite article.

In general, the invention compositions are characterized by high glass transition temperatures on the order of 180 °C or greater, high modulus (400 ksi or greater), good hot/wet retention of modulus (350 °C and higher), fracture toughness on the order of 1000 psi in 1/2 or greater, and low water absorption.

Example 1

This example illustrates the preparation of the diglycidyl ether component of the invention composition. Into a 5 l three-necked round botton flask, fitted with a stirrer, condenser, temperature probe, addition funnel, and nitrogen inlet was charged 500 g of 9,9-bis(4-hydroxyphenyl)fluorene [prepared as described by S.C. Lin and E.M. Pearce, J. Polym. Sci., Polym. Chem. Ed., 17, 3095-3119 (1979)], 1260 g isopropyl

alcohol, 236 g water, and 1573.4 g epichlorohydrin. While stirring, the mixture was heated to 70 °C and 476 g of 20% weight sodium hydroxide solution was added over ninety minutes. After the reaction mixture was held an hour at 74 °C, stirring was stopped and the reaction mixture allowed to separate. Then an aliquot of the water layer was removed and per cent base determined to be below 0.2% weight sodium hydroxide. A second addition of 219.7 g 20% sodium hydroxide was then made over an hour period while the reaction mixture was held at 74 °C. After holding at 74 °C for an hour and 45 minutes stirring was stopped, the mixture allowed to separate, and an aliquot of the water was removed and per cent base determined to be below 0.2% weight sodium hydroxide.

The upper layer was separated and the epichlorohydrin, isopropyl alcohol, and water removed under vacuum at 100 °C. The residue was dissolved in methylisobutyl ketone and charged back into the reaction flask. Then 1600 g of 5% weight sodium hydroxide was charged to the flask and the mixture held at 90 °C for 4 hours. The water layer was removed and the organic layer was washed with four 900 ml portions of 93 °C water. The product was recovered by removing the solvent under vacuum at 165 °C. The yield was 609 g of resin (94.1% of theory) having an epoxide equivalent weight (EEW) of 253.

Example 2

This example shows the properties of a diglycidyl ether cured with a stoichiometric amount of a polyaromatic polyamine curing agent. 50.0 g (0.196 equiv.) of the diglycidylether of 9,9-bis (4-hydroxyphenyl)fluorene was charged into a 150 ml beaker which was placed in 150 °C oven until the resin had melted. To this melt was added 19.6 g (0.196 equiv.) of α,α'-bis(3,5-dimethyl-4-aminophenyl)-p-diisopropylbenzene (EPON HPT(R) Curing Agent 1062), which had been melted in another oven at 170 °C. The resulting mixture was stirred vigorously by hand, degassed in a hot vacuum oven, and poured into a preheated (150 °C) glass mold. The resulting casting was cured for 2 hours at 150 °C and for 4 hours at 200 °C.

The cured casting was machined into test specimens which were used to obtain fracture toughness (Kq/ASTM) E399), flexural properties at room dry temperature and 93 °C/wet (ASTM D790), moisture absorption, and dynamic mechanical properties (ASTM D4056). The result of the tests are summarized in Table 1.

Example 3

This example shows the properties of a diglycidyl ether cured with an excess stoichiometric amount of a polyaromatic polyamine curing agent. The procedure of Example 1 was repeated using 45.0 g (0.180 equiv.) of the diglycidylether of 9,9-bis(4-hydroxyphenyl)fluorene and 25.8 g (0.258 equiv.) of α,α'-bis-(3,5-dimethyl-4-aminophenyl)-p-diisopropylbenzene.

The results of the tests are summarized in Table 1.

Example 4

The following examples show the properties of an elastomer modified diglycidyl ether/amine composition according to the invention. 50.0 g (0.196 equiv.) of the diglycidylether of 9,9-bis (4-hydroxyphenyl)-fluorene was charged into a 150 ml beaker which was placed in 150 °C oven until the resin had melted. Into this melt was added with vigorous stirring 10.0 g (0.00250 equiv.) of Hycar Carboxy Terminated (CTBN) Liquid Polymer 1300X8 (B.F. Goodrich Company) and 0.25 g tetrabutyl-ammonium bromide. The reaction mixture was held at 150 °C for 2 hours during which time it was stirred by hand every few minutes. To this was added 19.4 g (0.194 equiv.) of α,α'-bis(3,5-dimethyl-e-aminophenyl)-p-diisopropylbenzene which had been melted in another oven at 170 °C. The resulting mixture was stirred vigoursly by hand, degassed in a hot vacuum oven, and poured into a preheated (150 °C) glass mold. The resulting casting was cured for 2 hours at 150 °C and for 4 hours at 200 °C.

The cured casting was machined into test specimens which were used to obtain fracture toughness (Kq/ASTM E399), flexural properties at room dry temperature and 93 °C/wet (ASTM D790), moisture absorption, and dynamic mechanical properties (ASTM D4056). The result of the tests are summarized in Table 1.

Example 5

The procedure of Example 3 was repeated using 45.0 g (0.180 equiv.) of the diglycidylether of 9,9-bis(4-hydroxyphenyl)fluorene, 4.50 g (0.00225 equiv.) of Hycar Carboxy Terminated (CT) Liquid Polymer, 0.231 g tetrabutylammonium bromide, and 25.7 g (0.257 equiv.) of α,α'-bis(3,5-dimethyl-4-aminophenyl)-p-diisopropylbenzene.

The results of the tests are summarized in Table 1.

Examples 6-8

The procedure of Example 3 was repeated using 50 g (0.196 equiv.) of the diglycidylether of 9,9-(4-hydroxyphenyl)fluorene modified with 2.50 g (0.00125 equiv.), 7.70 g (0.00375 equiv.) and 10.0 g (0.00500 equiv.) of Hycar Carboxy Terminate (CT) Liquid Polymer 1300X8, and 25.9 g (0.0259 equiv.), 25.5 g (0.0225 equiv.) and 25.3 g (0.0253 equiv.) of α,α'-bis(3,5-dimethyl-4-aminophenyl)-p-diisopropylbenzene, respectively. The results of the test are summarized in Table 2.

TABLE 1

| Properties | Example 2 | Example 3 | Example 4 | Example 5 |
|---|---|---|---|---|
| Tg (TAN δ), °C | 299 | 189 | 236 | 179 |
| FLEX PROPERTIES (DRY) 1/ | | | | |
| Strength, $10^3$.kPa (ksi) | 152(22.0) | 125(18.2) | 132(19.2) | 130(18.8) |
| Modulus, $10^3$.kPa (ksi) | 3650(530.0) | 3450(501.1) | 3260(473.0) | 3240(470.6) |
| Elong, % | 6.7 | 4.4 | 6.5 | 5.6 |
| FLEX PROPERTIES (WET) 2/ | | | | |
| Strength, $10^3$.kPa (ksi) | 97.8(14.2) | 91.0(13.2) | 80.0(11.6) | 82.0(11.9) |
| Modulus, $10^3$.kPa (ksi) | 2890(419.1) | 2960(429.1) | 2430(353.3) | 2735(397.2) |
| Elong, % | 4.0 | 3.7 | 4.6 | 4.2 |
| FRACTURE TOUGHNESS | | | | |
| Kq, kPa√m (PSI.IN 1/2) | 501(455) | 669(608) | 890(819) | 1390(1263) |
| MOISTURE GAIN, % | 1.2 | 1.0 | 1.4 | 1.1 |

1/ Room temperature

2/ Tested in water @ 93 °C after two weeks immersion @ 93 °C

TABLE 2

| Properties | Example 6 | Example 7 | Example 8 |
| --- | --- | --- | --- |
| Tg (TAN δ), °C | 195 | 190 | 185 |
| FLEX PROPERTIES (DRY) 1/ | | | |
| Strength, $10^3$.kPa (ksi) | 134(19.3) | 117(17.0) | 114(16.6) |
| Modulus, $10^3$.kPa (ksi) | 3180(461.4) | 2890(419.4) | 2720(394.9) |
| Elong, % | 5.3 | 5.9 | 6.7 |
| FLEX PROPERTIES (WET) 2/ | | | |
| Strength, $10^3$.kPa (ksi) | 104(15.1) | 82.0(11.9) | 71.0(10.3) |
| Modulus, $10^3$.kPa (ksi) | 3130(453.7) | 2400(348.2) | 2200(320.5) |
| Elong, % | 4.8 | 4.7 | 4.7 |
| FRACTURE TOUGHNESS | | | |
| Kq, kPa.$\sqrt{m}$ (PSI.IN 1/2) | 967(879) | 1669(1517) | 2025(1841) |
| MOISTURE GAIN, % | 1.1 | 1.1 | 1.1 |

1/ Room temperature

2/ Tested in water @ 93 °C after two weeks immersion @ 93 °C

**Claims**

**Claims for the following Contracting States : BE, DE, FR, GB, IT, NL, SE**

**1.** A composition comprising
(a) a diglycidyl ether of 9,9-bis(4-hydroxyphenyl)fluorene;
(b) a normally liquid elastomeric polydiene comprising carboxyl and/or amine groups and having a molecular weight of from 2000 to 10000; and
(c) a polyaromatic polyamine curing agent for the diglycidyl ether, present in a stoichiometric excess, with respect to the diglycidyl ether, of at least 20 per cent.

**2.** A composition as claimed in claim 1 in which the polyaromatic polyamine is present in an amount of from 1.25 to 1.5 equivalents per equivalent of the diglycidyl ether.

**3.** A composition as claimed in claim 1 or 2 in which the polydiene elastomer is present in an amount of from 3 to 40 weight per cent, based on the weight of the diglycidyl ether.

4. A composition as claimed in any of claims 1 to 3 in which the elastomeric polymer is a carboxy or amine terminated polydiene.

5. A composition as claimed in any of claims 1 to 4 in which the elastomeric polydiene is present in the composition in an amount of from 5 to 25 weight per cent, based on the weight of the diglycidyl ether.

6. A composition as claimed in any of claims 1 to 5 in which the polyaromatic polyamine has the formula

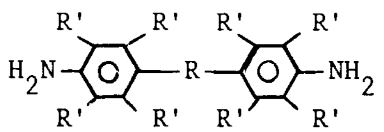
,

in which R is selected from alkaryl moieties, $-CH_2$, $-C(CH_3)_2$, $-O-$, $-SO_2-$, or $-S-$, and each R' is selected from H, lower alkyl and halide.

7. A composition as claimed in claim 6 in which R is

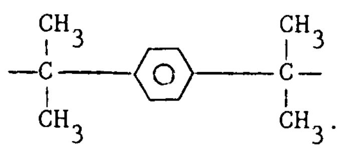

8. A composition as claimed in claim 6 in which the polyaromatic polyamine curing agent is a mixture of

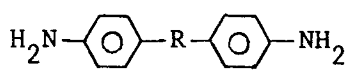

in which R is

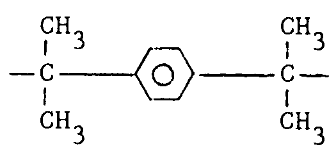

and

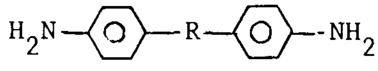

in which R is $-SO_2-$.

9. A cured composition resulting upon the curing of a curable composition as claimed in any one of claims 1 to 8.

**Claims for the following Contracting State : ES**

1. A process for curing a composition comprising
   (a) a diglycidyl ether of 9,9 – bis(4 – hydroxyphenyl)fluorene;
   (b) a normally liquid elastomeric polydiene comprising carboxyl and/or amine groups and having a molecular weight of from 2000 to 10000; and
   (c) a polyaromatic polyamine curing agent for the diglycidyl ether, present in a stoichiometric excess, with respect to the diglycidyl ether, of at least 20 per cent.

2. A process as claimed in claim 1 in which the polyaromatic polyamine is present in an amount of from 1.25 to 1.5 equivalents per equivalent of the diglycidyl ether.

3. A process as claimed in claim 1 or 2 in which the polydiene elastomer is present in an amount of from 3 to 40 weight per cent, based on the weight of the diglycidyl ether.

4. A process as claimed in any of claims 1 to 3 in which the elastomeric polymer is a carboxy or amine terminated polydiene.

5. A process as claimed in any of claims 1 to 4 in which the elastomeric polydiene is present in the composition in an amount of from 5 to 25 weight per cent, based on the weight of the diglycidyl ether.

6. A process as claimed in any of claims 1 to 5 in which the polyaromatic polyamine has the formula

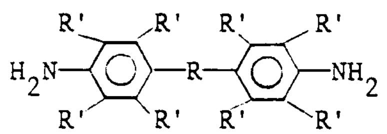 ,

   in which R is selected from alkaryl moieties, $-CH_2$, $-C(CH_3)_2$, $-O-$, $-SO_2-$, or $-S-$, and each R' is selected from H, lower alkyl and halide.

7. A process as claimed in claim 6 in which R is

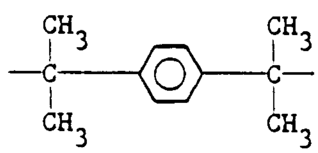 .

8. A process as claimed in claim 6 in which the polyaromatic polyamine curing agent is a mixture of

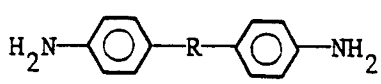

   in which R is

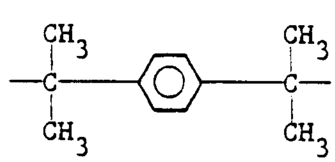

   and

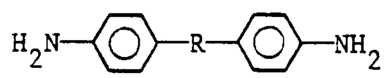

in which R is $-SO_2-$.

**Patentansprüche**
**Patentansprüche für folgende Vertragsstaaten : BE, DE, FR, GB, IT, NL, SE**

1. Eine Zusammensetzung, umfassend:
   a) einen Diclycidylether von 9,9-Bis(4-hydroxyphenyl)fluoren;
   b) ein unter Normalbedingungen flüssiges elastomeres Polydien, das Carboxyl- und/oder Aminogruppen enthält und ein Molekulargewicht im Bereich von 2000 bis 10000 aufweist; und
   c) ein polyaromatisches Polyaminhärtungsmittel für den Diglycidylether, das in Bezug auf den Diglydicylether in einem stöchiometrischen Überschuß von mindestens 20 % vorliegt.

2. Eine Zusammensetzung, wie in Anspruch 1 beansprucht, in der das polyaromatische Polyamin in einer Menge von 1,25 bis 1,5 Äquivalenten je Äquivalent des Diglycidylethers vorliegt.

3. Eine Zusammensetzung, wie in Anspruch 1 oder 2 beansprucht, in der das Polydienelastomer in einer Menge von 3 bis 40 Gew.-%, bezogen auf das Gewicht des Diglycidylethers, vorliegt.

4. Eine Zusammensetzung, wie in irgendeinem der Ansprüche 1 bis 3 beansprucht, in der das elastomere Polymer ein Polydien mit Carboxy- oder Aminoendgruppen ist.

5. Eine Zusammensetzung, wie in irgendeinem der Ansprüche 1 bis 4 beansprucht, in der das elastomere Polydien in der Gesamtzusammensetzung in einer Menge von 5 bis 25 Gew.-%, bezogen auf das Gewicht des Diglycidylethers, vorliegt.

6. Eine Zusammensetzung, wie in irgendeinem der Ansprüche 1 bis 5 beansprucht, in der das polyaromatische Polyamin die nachstehende Formel

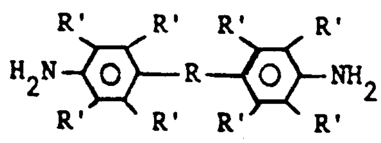

aufweist, in der R ausgewählt ist aus Alkarylbausteinen, $-CH_2$, $-C(CH_3)_2$, $-O-$, $-SO_2$ oder $-S-$, und jedes R' ausgewählt ist aus H, niedrigem Alkyl und einem Halogenid.

7. Eine Zusammensetzung, wie in Anspruch 6 beansprucht, in der R der nachstehenden Formel entspricht:

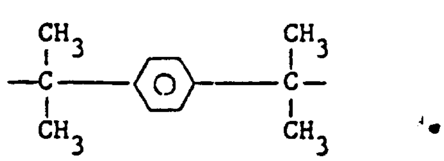

8. Eine Zusammensetzung, wie in Anspruch 6 beansprucht, in der das polyaromatische Polyaminhärtungsmittel eine Mischung aus einer Verbindung der Formel

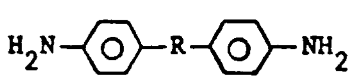,

in der R die Bedeutung

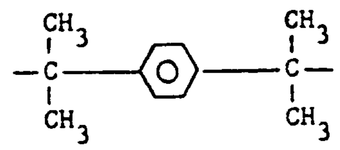

hat,
und einer Verbindung der Formel

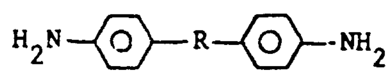

ist, in der R die Gruppe $-SO_2-$ ist.

9. Eine gehärtete Zusammensetzung, die beim Härten einer härtbaren Zusammensetzung, wie in irgendeinem der Ansprüche 1 bis 8 beansprucht, gebildet wird.

**Patentansprüche für folgenden Vertragsstaat : ES**

1. Ein Verfahren zum Härten einer Zusammensetzung, umfassend:
   a) einen Diclycidylether von 9,9-Bis(4-hydroxyphenyl)fluoren;
   b) ein unter Normalbedingungen flüssiges elastomeres Polydien, das Carboxyl- und/oder Aminogruppen enthält und ein Molekulargewicht im Bereich von 2000 bis 10000 aufweist; und
   c) ein polyaromatisches Polyaminhärtungsmittel für den Diglycidylether, das in Bezug auf den Diglydicylether in einem stöchiometrischen Überschuß von mindestens 20 % vorliegt.

2. Ein Verfahren, wie in Anspruch 1 beansprucht, in dem das polyaromatische Polyamin in einer Menge von 1,25 bis 1,5 Äquivalenten je Äquivalent des Diglycidylethers vorliegt.

3. Ein Verfahren, wie in Anspruch 1 oder 2 beansprucht, in dem das Polydienelastomer in einer Menge von 3 bis 40 Gew.-%, bezogen auf das Gewicht des Diglycidylethers, vorliegt.

4. Ein Verfahren, wie in irgendeinem der Ansprüche 1 bis 3 beansprucht, in dem das elastomere Polymer ein Polydien mit Carboxy- oder Aminoendgruppen ist.

5. Ein Verfahren, wie in irgendeinem der Ansprüche 1 bis 4 beansprucht, in dem das elastomere Polydien in der Gesamtzusammensetzung in einer Menge von 5 bis 25 Gew.-%, bezogen auf das Gewicht des Diglycidylethers, vorliegt.

6. Ein Verfahren, wie in irgendeinem der Ansprüche 1 bis 5 beansprucht, in dem das polyaromatische Polyamin die nachstehende Formel

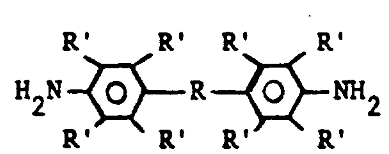

aufweist, in der R ausgewählt ist aus Alkarylbausteinen, $-CH_2$, $-C(CH_3)_2$, $-O-$, $-SO_2$ oder $-S-$, und jedes R' ausgewählt ist aus H, niedrigem Alkyl und einem Halogenid.

**7.** Ein Verfahren, wie in Anspruch 6 beansprucht, in dem R der nachstehenden Formel entspricht:

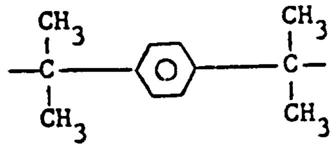

.

**8.** Ein Verfahren, wie in Anspruch 6 beansprucht, in dem das polyaromatische Polyaminhärtungsmittel eine Mischung aus einer Verbindung der Formel

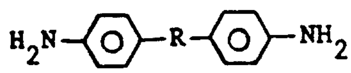

,

in der R die Bedeutung

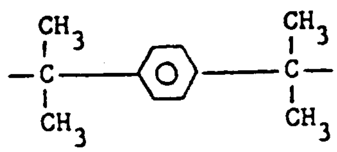

hat,
und einer Verbindung der Formel

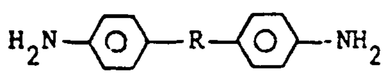

ist, in der R die Gruppe $-SO_2-$ ist.

**Revendications**
**Revendications pour les Etats contractants suivants : BE, DE, FR, GB, IT, NL, SE**

**1.** Une composition comprenant
(a) un éther diglycidique de 9,9-bis(4-hydroxyphényl)fluorène ;
(b) un polydiène élastomère normalement liquide comprenant des groupes carboxyle et/ou amine et ayant un poids moléculaire compris entre 2000 et 10 000 ; et
(c) un durcisseur polyamine polyaromatique pour l'éther diglycidique, présent en excès stoechiométrique d'au moins 20 pour cent par rapport à l'éther diglycidique.

**2.** Une composition selon la revendication 1, dans laquelle la polyamine polyaromatique est présente à raison de 1,25 à 1,5 équivalent par équivalent de l'éther diglycidique.

**3.** Une composition selon la revendication 1 ou 2, dans laquelle l'élastomère polydiène est présent à raison de 3 à 40 pour cent en poids, par rapport au poids de l'éther diglycidique.

**4.** Une composition selon l'une quelconque des revendications 1 à 3, dans laquelle le polymère élastomère est un polydiène à groupes terminaux carboxy ou amine.

**5.** Une composition selon l'une quelconque des revendications 1 à 4, dans laquelle le polydiène élastomère est présent dans la composition à raison de 5 à 25 pour cent en poids, par rapport au poids de l'éther diglycidique.

**6.** Une composition selon l'une quelconque des revendications 1 à 5, dans laquelle la polyamine polyaromatique a la formule

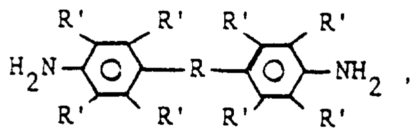

dans laquelle R est choisi parmi des portions alcaryle, $-CH_2$, $-C(CH_3)_2$, $-O-$, $-SO_2-$ ou $-S-$ et chaque R' est choisi parmi H et des groupes alcoyle inférieur et halogénure.

**7.** Une composition selon la revendication 6, dans laquelle R est

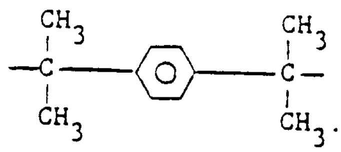

**8.** Une composition selon la revendication 6, dans laquelle le durcisseur polyamine polyaromatique est un mélange de

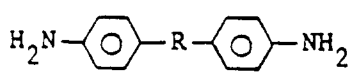

où R est

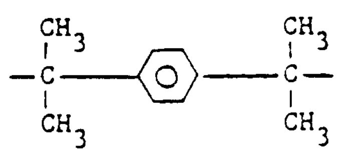

et de

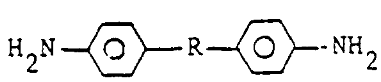

où R est $-SO_2-$.

**9.** Une composition durcie résultant du durcissement d'une composition durcissable selon l'une quelconque des revendications 1 à 8.

**Revendications pour l'Etat contractant suivant : ES**

**1.** Un procédé pour le durcissement d'une composition comprenant
(a) un éther diglycidique de 9,9-bis(4-hydroxyphényl)fluorène ;
(b) un polydiène élastomère normalement liquide comprenant des groupes carboxyle et/ou amine et ayant un poids moléculaire compris entre 2000 et 10 000 ; et
(c) un durcisseur polyamine polyaromatique pour l'éther diglycidique, présent en excès stoechiométrique d'au moins 20 pour cent par rapport à l'éther diglycidique.

2. Un procédé selon la revendication 1, dans lequel la polyamine polyaromatique est présente à raison de 1,25 à 1,5 équivalent par équivalent de l'éther diglycidique.

3. Un procédé selon la revendication 1 ou 2, dans lequel l'élastomère polydiène est présent à raison de 3 à 40 pour cent en poids.

4. Un procédé selon l'une quelconque des revendications 1 à 3, dans lequel le polymère élastomère est un polydiène à groupes terminaux carboxy ou amine.

5. Un procédé selon l'une quelconque des revendications 1 à 4, dans lequel le polydiène élastomère est présent dans la composition à raison de 5 à 25 pour cent en poids, par rapport au poids de l'éther diglycidique.

6. Un procédé selon l'une quelconque des revendications 1 à 5, dans lequel la polyamine polyaromatique a la formule

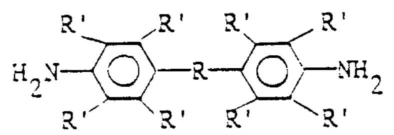

,

dans laquelle R est choisi parmi des portions alcaryle, $-CH_2$, $-C(CH_3)_2$, $-O-$, $-SO_2-$ ou $-S-$ et chaque portion R' est choisie parmi H et des groupes alcoyle inférieur et halogénure.

7. Un procédé selon la revendication 6, dans lequel R est

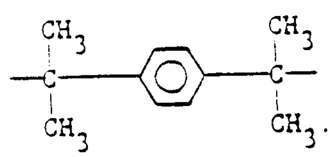

8. Un procédé selon la revendication 6, dans lequel le durcisseur polyamine polyaromatique est un mélange de

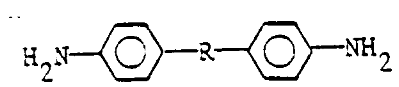

où R est

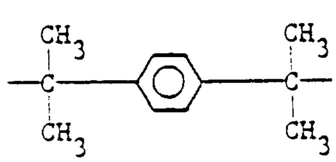

et de

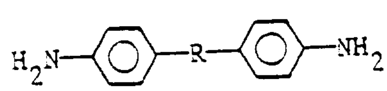

où R est $-SO_2-$.